# EUROPEAN PATENT APPLICATION

(11) **EP 0 903 137 A1**
(43) Date of publication of application: **24.03.1999**
(21) Application number: 98116722.4
(22) Date of filing: 03.09.1998
(51) Int. Cl.: A61F 13/20

(54) **Tampon and methods of making the same**

(30) Priority: 19.09.1997 US 934282
(71) Applicant: Hauni Richmond, Inc., Richmond, VA 23231 (US)
(72) Inventor: Preisner, Peter M., Quinton, VA 23141 (US); Oppe, Hans-Joachim, Midlothian, VA 23113 (US); McLean, William J., New Kent, VA 23124 (US)
(74) Representative: Herrmann, Günther

(57) **Abstract**

A tampon (1), wherein a compressed absorbent pledget (2) is confined in a foraminous envelope (3), is conveyed through an annular heating zone in which the envelope is at least partially bonded to the pledget so that the envelope shares the expansion of the pledget when the tampon is in use. The heating zone can be established by one or more circumferentially complete or composite heating elements. The bonding temperature is selected in dependency upon the materials of the pledget and the envelope. The conversion of a blank of thermoplastic coverstock into the envelope can precede the compression of the pledget, and such compression can precede the bonding step.

## Description

### BACKGROUND OF THE INVENTION

The invention relates to tampons, and more particularly to improvements in digital and applicator type catamenial tampons. Still more particularly, the invention relates to improvements in tampons of the type disclosed in commonly owned U.S. Patent No. 5,004,467 granted April 2, 1991 to Alfred Hinzmann et al. for "TAMPON". The disclosure of Hinzmann et al., and of each U.S. Patent referred to in Hinzmann et al., is incorporated herein by reference.

A drawback of presently known tampons is that the foraminous envelope (made of a blank of sheet-like material called coverstock) which surrounds the normally cylindrical or substantially cylindrical pledget of cotton, rayon or other suitable absorbent material is apt to become at least partially separated from the pledget, e.g., in actual use of the tampon. This might create problems in connection with the withdrawal of an expanded tampon from a body cavity, e.g., by means of the customary removal cord.

Another drawback of conventional tampons is that the envelope (such as a converted blank of sheet-like foraminous coverstock material) can become at least partially separated from the compressed pledget prior to actual use of the tampon, e.g., during insertion into or during expulsion from an applicator. This can create problems in connection with the manipulation of such tampons in the manufacturing plant and can give rise to an undesirably high percentage of rejects.

### OBJECTS OF THE INVENTION

An object of the invention is to enhance the quality, such as the stability, of digital and/or applicator-type catamenial tampons.

Another object of the invention is to ensure that all dimensional changes of the pledget are shared by the envelope for the pledget.

A further object of the invention is to provide a novel and improved method of treating a tampon, or a series of tampons, of the type wherein a compressed pledget is surrounded by an envelope (converted coverstock).

An additional object of the invention is to provide a novel and improved method of reliably confining an expansible pledget in an envelope constituting a converted blank of coverstock material.

Still another object of the invention is to provide an apparatus for the practice of the above outlined method of making and/or manipulating digital and/or applicator type tampons.

A further object of the invention is to provide an apparatus which can be readily installed in numerous types of existing production lines for catamenial tampons.

Another object of the invention is to provide a catamenial tampon which constitutes an improvement over and a further development of tampons disclosed in U.S. Patent No. 5,004,467 to Hinzmann et al.

An additional object of the invention is to provide a tampon which can be mass-produced at a reasonable cost and is ready for introduction into a body cavity or into an applicator.

### SUMMARY OF THE INVENTION

One feature of the present invention resides in the provision of a method of making a tampon from a deformable absorbent pledget - which is expansible in actual use of the tampon in a body cavity - and a deformable foraminous coverstock for the pledget. The pledget and the coverstock contain materials which are bondable to each other in response to the application of adequate heat, and the method comprises the steps of converting the coverstock into an envelope which at least partially surrounds the pledget, compressing the envelope and the pledget including exerting an adequate pressure against an external surface (e.g., a substantially cylindrical surface) of the envelope, and at least partially bonding the envelope to the pledget so that the envelope shares the expansion of the pledget in actual use of the tampon.

The converting step can precede the compressing and bonding steps, and one of the compressing and bonding steps can precede the other of these (compressing and bonding) steps.

At least a portion of one of the compressing and bonding steps can be carried out simultaneously with the other of these (compressing and bonding) steps.

At least two of the converting, compressing and bonding steps can be carried out simultaneously. It is presently preferred to select the sequence of steps in such a way that the compressing step precedes the bonding step and follows the converting step.

The method can further comprise the step of advancing the envelope (i.e., the converted coverstock) and the pledget therein along a predetermined path, and the bonding step of such method can include establishing at least one heating zone which at least partially surrounds a portion of such path. If the tampon employs a substantially cylindrical pledget having a central longitudinal axis, the advancing step can comprise moving the envelope and the pledget in the direction of such axis.

It is presently preferred to select the material of the pledget from a group consisting of cotton and rayon, and the material of the coverstock is preferably a suitable thermoplastic material, e.g., a material which is presently utilized for the making of conventional tampons.

The bonding step can include heating at least the envelope to a temperature of between about 225° and 350°F; if the pledget is made of cotton or rayon and the material of the coverstock is a standard thermoplastic material, the pledget and/or the envelope is preferably heated to a temperature of between about 275° and 300°F.

The method can further comprise the steps of advancing the envelope and the pledget therein along a first path subsequent to the compressing step on to a bonding station, thereupon advancing the envelope and the pledget therein along a second path through the bonding station, and thereafter advancing the tampon away from the second path along a third path; the bonding step is carried out at the aforesaid station.

Another feature of the invention resides in the provision of a method of processing a series of successive tampons of the type having an absorbent compressible pledget - which is expansible in actual use of the tampon - and a foraminous envelope at least partially confining the pledget. The pledgets and the envelopes contain materials which are bondable to each other in response to adequate heating, and the method comprises the steps of advancing the tampons of the series in a predetermined direction along a predetermined path, and at least partially bonding the envelopes of successive tampons of the series to the respective pledgets in at least one portion of the path so that each envelope shares the expansion of the respective pledget in actual use of the corresponding tampon.

The bonding step can include establishing at least one heating zone which at least partially surrounds the at least one portion of the path.

The method can further comprise the steps of delivering successive tampons of the series along a second path to a first portion of the predetermined path, and advancing successive tampons of the series from a second portion of the predetermined path downstream of the first portion (as seen in the predetermined direction) and subsequent to the bonding step.

The bonding step can comprise heating at least the envelopes of successive tampons of the series of tampons to a temperature of between about 225° and 350°F, preferably between about 275° and 300°F if the pledgets are made of cotton or rayon and the coverstock is made of a standard thermoplastic material.

A further feature of the invention resides in the provision of a tampon which comprises a compressed pledget which is expansible in actual use of the tampon, and a foraminous envelope which at least partially surrounds and is bonded to the pledget.

The envelope is or can be heat sealed to the pledget. The arrangement can be such that the envelope closely follows at least a major portion of the external surface of the pledget.

As already mentioned above, the pledget can be made, at least in part, of rayon and/or cotton, and the envelope can be made of a sheet-like foraminous thermoplastic material which can be readily heat sealed to the pledget.

The improved tampon preferably further comprises a customary removal cord which is connected at least to the pledget and extends from the envelope.

The novel features which are considered as characteristic of the invention are set forth in particular in the appended claims. The improved tampon itself, however, both as to its construction and the methods of making the same, together with numerous additional important and advantageous features thereof, will be best understood upon perusal of the following detailed description of certain presently referred specific embodiments with reference to the accompanying drawings.

### BRIEF DESCRIPTION OF THE DRAWINGS

FIG. 1 is a perspective view of a partly finished tampon;
FIG. 2 is a similar perspective view of a modified partly finished tampon;
FIG. 3 is a similar perspective view of a third partly finished tampon;
FIG. 4 is a perspective view of a tampon as it appears upon compression of the partly finished tampon shown in FIG. 1, 2 or 3;
FIG. 5 is a perspective view of a tampon constituting a modification of the tampon shown in FIG. 4;
FIG. 6 is a perspective view of a structure which can convert partly finished tampons similar to those shown in FIG. 4 or 5 into tampons embodying the present invention;
FIG. 7 is a fragmentary perspective view of an apparatus which embodies the structure of FIG. 6; and
FIG. 8 is a similar fragmentary perspective view of an apparatus constituting a modification of the apparatus shown in FIG. 7.

### DESCRIPTION OF PREFERRED EMBODIMENTS

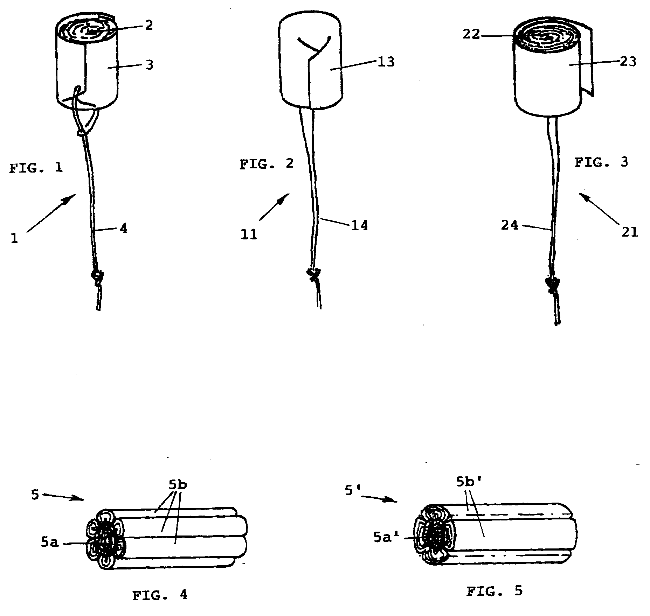

FIG. 1 shows a partially finished tampon 1 comprising an at least substantially cylindrical pledget 2, an at least substantially cylindrical envelope 3 (converted blank of coverstock), and a flexible removal cord or drawstring 4. The tampon 1 of FIG. 1 corresponds to that shown in FIG. 1 of U.S. Patent No. 5,004,467 and can be produced in a manner as disclosed in the patent. The cord 4 can be installed and configurated in a manner as disclosed in U.S. Patent No. 4,836,587 granted June 6, 1989 to Alfred Hinzmann and referred to in U.S. Patent No. 5,004,467. The envelope 3 is open at the end which is remote from the cord 4, and the latter forms a loop which extends through the cylindrical wall of the envelope 3 as well as substantially diametrically through the adjacent (confined) end portion of the pledget 2.

FIG. 2 shows a modified partially finished tampon 11 having a cylindrical pledget (confined in the envelope), a cylindrical envelope 13, and a removal cord 14. The latter is anchored in the adjacent end portion of the pledget and extends through and beyond the adjacent open end of the envelope 13. The cord 14 can be anchored in the pledget of the tampon 11 prior to confinement of the pledget in the envelope (converted blank of coverstock) 13; on the other hand, the cord 4 of the tampon 1 must be anchored in the respective end portion of this tampon subsequent to confinement of the pledget 2 in the envelope 3, i.e., subsequent to conversion of a blank of thermoplastic coverstock into the envelope 3.

FIG. 3 illustrates a partially finished tampon 21 having a cylindrical pledget 22, an open-ended cylindrical envelope 23, and a removal cord or drawstring 24 which is anchored in the pledget 22 and extends through and beyond the respective open end of the envelope 23. In contrast to the envelopes 3 and 13, which can constitute converted blanks of the type shown at 112 in FIG. 4 of U.S. Patent No. 5,004,467, the envelope 23 is a converted elongated strip- or web-shaped blank which is convoluted around the cylindrical portion of the external surface of the pledget 22.

FIG. 4 shows a tampon 5 (with the removal cord omitted) which is obtained as a result of compression of the partially finished tampon 1, 11 or 21. The compressing step normally involves the application of radially inwardly directed force against the cylindrical portion of the external surface of the envelope 3 or 13, or the application of radially inwardly directed force against the only accessible (cylindrical external) surface, of the envelope 23. The tampon 5 of FIG. 4 includes a cylindrical central portion or core 5a surrounded by six axially parallel uniformly distributed substantially cylindrical (rod-shaped) projections or ribs 5b. In accordance with heretofore known proposals, the tampon 5 of FIG. 4 is ready for use or for confinement in a suitable applicator, e.g., an applicator of the type disclosed in U.S. Patent No. 4,755,164 granted July 5, 1988 to Alfred Hinzmann and referred to in U.S. Patent No. 5,004,467.

FIG. 5 shows a modified tampon 5' (with the removal cord omitted) which can be obtained as a result of compression of the partially finished tampon 1, 11 or 21. The difference between the tampons 5 (FIG. 4) and 5' (FIG. 5) is that the tampon 5' comprises fewer (such as four) projections or ribs 5b' surrounding a centrally located elongated core 5a'.

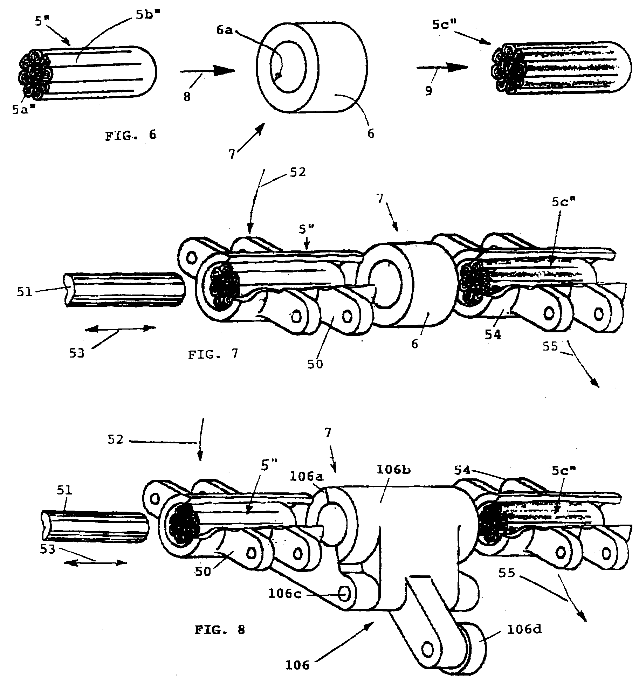

Referring to FIG. 6, there is shown a portion of an apparatus which can be utilized for the practice of one embodiment of our improved method. A tampon 5" having a compressed pledget closely surrounded by a deformed envelope, and a cord (not shown) includes a centrally located elongated core 5a" surrounded by eight axially parallel equidistant projections or ribs 5b". The character 6 denotes a schematically shown circumferentially complete annular heating or bonding element or tool which is installed at a heating or bonding zone or station 7 and surrounds a portion of an elongated path (denoted by the arrows 8, 9) for axial advancement of the tampons 5", e.g., under the action of a reciprocable pusher or plunger (such as the pusher or plunger 51 shown in FIG. 7). The element 6 heats at least the envelope of the tampon 5" to a temperature at which the envelope is at least partially bonded to the adjacent portion of the compressed pledget forming part of the tampon 5". The thus obtained finished tampon 5c" is ready for use or for insertion into an applicator. Since the envelope is bonded (at least in part) to the adjacent portion(s) of the pledget forming part of the finished tampon 5c", such envelope is highly unlikely to become separated from the compressed pledget in actual use and/or during introduction into an applicator and/or during expulsion from an applicator into a body cavity.

If the pledget of the tampon 5" is made of cotton or rayon, and if the envelope of such tampon is a converted (deformed) blank of customary sheet-like or strip-shaped thermoplastic coverstock material, the heating element or tool 6 can be maintained at a temperature which heats at least the envelope of the tampon 5" to a temperature of between about 225° and 350°F, particularly between about 275° and 300°F. Such temperature ranges have been found to be highly satisfactory to ensure the adherence of the deformed envelope to the compressed pledget during making and during manipulation of the tampon 5c", during storage (e.g., in an applicator), during introduction into a body cavity (either digitally or by resorting to an applicator), as well as during extraction of an expanded tampon by way of its cord or drawstring.

The illustrated heating element or tool 6 has a smooth cylindrical internal surface 6a. However, it is also possible to replace the surface 6a with a surface which more closely conforms to the outlines (exposed or accessible portions) of the ribs 5b" of successive tampons 5" which are caused to advance axially along their path (arrows 8, 9) through the modified heating element at the bonding station or zone 7. Such modified (splined) configuration of the internal surface of the heating element at the bonding station 7 even more reliably ensures adequate adherence or bonding of the envelope of the tampon 5" to the respective pledget.

The exact manner (e.g., electrically) in which the element 6 or an equivalent heating element is maintained at a requisite temperature forms no part of the present invention. It is to be understood that the apparatus for the manipulation (heating) of tampons 5" can employ any suitable heating means the action of which can be properly regulated to ensure that the pledget and/or the envelope of each tampon 5" is heated to a desired temperature at which the thus established bond between the envelope and the pledget is sufficiently reliable to prevent premature or untimely separation of the envelope from the compressed pledget, not only in the machine or production line which turns out tampons 5c" but also in storage (e.g., in applicators) as well as in actual use.

It is further possible to design the heating element at the station 7 in such a way that it serves as a means for draping blanks (e.g., blanks of the type shown in FIG. 4 of U.S. Patent No. 5,004,667) around uncompressed pledgets and/or for carrying out the draping or converting operation simultaneously with requisite radial compression of the pledget. Thus, at least two of the blank converting, pledget compacting and bonding steps can be carried out simultaneously. For example, the heating element 6 of FIG. 6 can be incorporated into or combined with the folding die shown in FIG. 4 of U.S. Patent No. 5,004,467.

FIG. 7 shows a portion of an apparatus which includes the structure of FIG. 6 and is designed to convert a series of successive tampons 5" into finished tampons 5c". The apparatus comnprises a first conveyor including an endless chain having links 50 (only one shown) arranged to orbit (continuously or stepwise) along an endless path in the direction indicated by an arrow 52. The links 50 have receptacles serving to advance successive tampons 5" of the series into temporary register with the central opening of the heating element or tool 6 at the bonding station or zone 7 as well as with the aforementioned plunger or pusher 51 which is operated (e.g., by a cam, by a hydraulic or pneumatic cylinder and piston unit or in any other suitable way) to reciprocate in directions indicated by a double-headed arrow 53. The plunger 51 advances successive tampons 5" from the receptacles of successive chain links 50 through and beyond the annular heating element 6 at the bonding station 7, and also advances the thus obtained finished tampons 5c" into the receptacles of successive links 54 (only one shown in FIG. 7) which advance along an endless path (arrow 55) to deliver successive finished tampons 5c" to the next processing station, e.g., to the applicator charging or loading station, not shown.

It will be seen that the receptacles of successive links 54 accept successive finished tampons 5c" in a portion of the path for the advancement of tampons 5" under the action of the plunger 51 which is located downstream of the path portion serving to receive links 50 (as seen in the direction of advancement of the plunger 51 from the retracted position of FIG. 7 to an extended position in which the freshly finished tampon 5c" is located in the receptacle of the adjacent link 54. The plunger 51 can be designed and mounted and operated to perform uninterrupted strokes from the fully retracted to the fully extended position, or is caused to move intermittently so that a tampon 5" which advances from the receptacle of a link 50 to the receptacle of a link 54 is compelled to dwell in the heating element 6 for a given interval of time. This depends upon the heating action of the element 6 at the bonding station 7 as well as upon the characteristics of the materials of the envelopes and pledgets forming part of the tampons 5".

The apparatus of FIG. 8 is or can be identical with the apparatus of FIG. 7 save for the construction of the heating element or tool 106 at the bonding station 7. The illustrated heating element 106 has a fixed jaw 106a, a complementary second jaw 106b which can be a mirror image of the fixed jaw 106a and is pivotable about the axis of a shaft 106c, and means (including a roller follower 106d) for pivoting the jaw 106b between an open position (not shown) and the (illustrated) operative position. The jaws 106a, 106b cooperate to heat the pledgets and/or the envelopes of successive tampons 5" to a desired bonding temperature, and these jaws can further cooperate with each other to carry out the compressing step or to carry out an additional (secondary) compressing step. Thus, the jaws 106a, 106b can convert partially finished tampons 1, 11 or 21 into finished tampons 5c", or these jaws can effect a further compression (and simultaneous heating or bonding) of tampons 5, 5' or 5".

The plunger 51 of FIG. 8 is or can be operated to interrupt its movement from the fully retracted position of FIG. 8 to the fully extended position (in which a freshly obtained or formed tampon 5c" is located in the receptacle of the adjacent link 54) so that a tampon 5" comes to a halt within the fixed jaw 106a before the jaw 106b is pivoted to the illustrated closed or operative position. The jaw 106b is thereupon returned to the open position (or to a partly open position) and the plunger 51 resumes its axial movement to advance the freshly obtained tampon 5c" into the receptacle of the adjacent link 54.

The roller follower 106d can extend into an endless groove of a suitable rotary cam (not shown) which causes the jaw 106b to perform its pivotal movements in synchronism with the reciprocatory movements of the plunger 51, with the intermittent movements of the links 50 along their endless path (arrow 52), and with the intermittent movements of the links 54 along their endless path (arrow 55). The apparatus of FIG. 7 is even simpler than that of FIG. 8 because the apparatus of FIG. 7 employs a stationary one-piece heating element or tool 6.

The illustrated apparatus and the aforedescribed methods are susceptible of numerous additional modifications without departing from the spirit of the present invention. For example, the links 50 of the first chain conveyor and/or the links 54 of the second chain conveyor can serve as a means for compressing partially finished tampons of the type shown in FIG. 1, 2 or 3. Thus, the compressing or compacting step can be carried out prior to, simultaneously with, or subsequent to the heating or bonding step. Furthermore, the links 50 can serve the additional function of converting blanks of coverstock into envelopes which at least partially surround the respective pledgets (i.e., the links 50 can perform the function of folding dies of the type shown (at 14) in (FIG. 4 of) U.S. Patent No. 5,004,467. In fact, it is even possible to employ a heating element (such as 6) as a means for converting blanks into envelopes, for simultaneously deforming the envelopes with attendant compression of the respective pledgets, and to simultaneously carry out the bonding operation.

It is also possible to provide the envelope-contacting surfaces of the jaws 106a, 106b with suitable profiles to alter the external shapes of successively delivered tampons 5" or to ensure that the internal surfaces of the jaws 106a, 106b come into larger-area contact with the adjacent portions of external surfaces of successive tampons 5" in response to each closing of the composite heating element 106.

An important advantage of the improved methods, apparatus and tampons 5c" is that the envelopes become integral parts of the respective pledgets so that the envelopes are compelled to share the expansion of the pledgets when such pledgets are caused to gather moisture. This simplifies the manipulation of the tampons in the manufacturing plant as well as in actual use regardless of whether or not the tampons 5c" are to be confined in suitable applicators.

Without further analysis, the foregoing will so fully reveal the gist of the present invention that others can, by applying current knowledge, readily adapt it for various applications without omitting features that, from the standpoint of prior art, fairly constitute essential characteristics of the generic and specific aspects of our contribution to the art of tampons and methods of making the same and, therefore, such adaptations should and are intended to be comprehended within the meaning and range of equvalence of the appended claims.

## Claims

1. A method of making a tampon from a deformable absorbent pledget - which is expansible in actual use of the tampon - and a deformable foraminous coverstock for the pledget, said pledget and said coverstock containing materials which are bondable to each other in response to heating, comprising the steps of converting the coverstock into an envelope which at least partially surrounds the pledget; compressing the envelope and the pledget including exerting a pressure against an external surface of the envelope; and at least partially bonding the envelope to the pledget so that the envelope shares the expansion of the pledget in actual use of the tampon.

2. The method of claim 1, wherein said converting step precedes said compressing and bonding steps and one of said compressing and bonding steps precedes the other of said compressing and bonding steps.

3. The method of claim 1, wherein at least a portion of one of said compressing and bonding steps is carried out simultaneously with at least a portion of the other of said compressing and bonding steps.

4. The method of claim 1, wherein at least two of said converting, compressing and bonding steps are carried out simultaneously.

5. The method of claim 1, wherein said compressing step precedes said bonding step and follows said converting step.

6. The method of claim 1, further comprising the step of advancing the envelope and the pledget along a predetermined path, said bonding step including establishing at least one heating zone at least partially surrounding a portion of said path.

7. The method of claim 6 of making a tampon including a substantially cylindrical pledget having an axis, wherein said advancing step includes moving the envelope and the pledget in the direction of said axis.

8. The method of claim 1, wherein the material of the pledget is selected from the group consisting of cotton and rayon.

9. The method of claim 1, wherein the material of the coverstock is a thermoplastic material.

10. The method of claim 1, wherein said bonding step includes heating the envelope to a temperature of between about 225° and 350°F.

11. The method of claim 1, wherein said bonding step includes heating the envelope to a temperature of between about 275° and 300°F.

12. The method of claim 1, further comprising the steps of advancing the envelope and the pledget along a first path subsequent to said compressing step to a bonding station, thereupon advancing the envelope and the pledget along a second path through the bonding station, and advancing the tampon away from said second path along a third path, said bonding step being carried out at said station.

13. A method of processing a series of successive tampons of the type having an absorbent compressible pledget - which is expansible in actual use of the tampon - and a foraminous envelope at least partially confining the pledget, said pledgets and said envelopes containing materials which are bondable to each other in response to heating, comprising the steps of advancing the tampons of said series in a predetermined direction along a predetermined path; and at least partially bonding the envelopes of successive tampons of said series to the respective pledgets in at least one portion of said path so that each envelope shares the expansion of the respective pledget in actual use of the corresponding tampon.

14. The method of claim 13, wherein said bonding step includes establishing at least one heating zone at least partially surrounding said at least one portion of said path.

15. The method of claim 13, further comprising the steps of delivering successive tampons of said series along a second path to a first portion of said predetermined path, and advancing successive tampons of said series from a second portion of said predetermined path downstream of said first portion, as seen in said direction, and subsequent to said bonding step.

16. The method of claim 13, wherein said bonding step comprises heating the envelopes of successive tampons of said series to a temperature of between about 225° and 350°F.

17. The method of claim 13, wherein said bonding step comprises heating the envelopes of successive tampons of said series to a temperature of between about 275° and 300°F.

18. The method of claim 13, wherein the material of said pledgets is selected from the group consisting of cotton and rayon.

19. The method of claim 13, wherein the material of said envelopes is a thermoplastic material.

20. A tampon comprising a compressed pledget which is expansible in actual use of the tampon; and a foraminous envelope at least partially surrounding and bonded to said pledget.

21. The tampon of claim 20, wherein the envelope is heat sealed to the pledget.

22. The tampon of claim 20, wherein the envelope closely follows at least a major portion of an external surface of the pledget.

23. The tampon of claim 20, wherein the material of the pledget is selected from the group consisting of cotton and rayon.

24. The tampon of claim 20, further comprising a removal cord connected at least to said pledget and extending from said envelope.
